# FASCICULE DE BREVET EUROPEEN

(11) **EP 3 930 673 B1**
(45) Date de publication et mention de la délivrance du brevet: **09.12.2026**
(21) Numéro de dépôt: 20723450.1
(22) Date de dépôt: 26.02.2020
(51) Int. Cl.: A61Q 19/00, A61K 8/34, A61K 8/44, A61K 8/73, A61K 8/97

(54) **COMPOSITION COSMÉTIQUE À BASE DE PRODUIT VÉGÉTAL**
KOSMETISCHE ZUSAMMENSETZUNG AUF DER BASIS EINES PFLANZENPRODUKTS
COSMETIC COMPOSITION BASED ON A PLANT PRODUCT

(30) Priorité: 26.02.2019 FR 1901973
(43) Date de publication de la demande: 05.01.2022
(73) Titulaire: Freedge SAS, 92210 Saint-Cloud (FR)
(72) Inventeur: CAISEY, Laurence, 94320 THIAIS (FR); CABANNES, Magali, 63100 CLERMONT FERRAND (FR); POULET, Lucille, 63100 CLERMONT FERRAND (FR); BERTHON, Jean-Yves, 63540 ROMAGNAT (FR); GUYON, Fabrice, 92210 SAINT CLOUD (FR); MARTIN, Arnaud, 21000 DIJON (FR)
(74) Mandataire: Yes My Patent
(86) Numéro de dépôt international: PCT/FR2020/000047
(87) Numéro de publication internationale: WO 2020/174133

(56) Documents cités:
- FR-A1- 2 961 693
- FR-A1- 2 968 551
- FR-A1- 3 032 614
- US-A1- 2018 085 303
- MBARKI N: "Mélanger le citron et les pommes de terre pour éliminer les taches du visage - NewsMAG", NEWSMAG, 29 October 2019 (2019-10-29), XP093100116, Retrieved from the Internet <URL:https://www.newsmag.tn/melanger-le-citron-et-les-pommes-de-terre-pour-eliminer-les-taches-du-visage/> [retrieved on 20231110]

## Description

### DOMAINE DE L'INVENTION

La présente invention concerne une composition comprenant au moins un produit végétal, et au moins un agent texturant, un kit de fabrication d'une composition cosmétique comprenant ces ingrédients, ainsi qu'un procédé de fabrication d'une composition cosmétique, et l'utilisation de ladite composition cosmétique pour purifier, hydrater et/ou renforcer l'éclat de la peau.

Aujourd'hui, que ce soit dans le secteur de l'alimentaire ou le secteur cosmétique, une attention toute particulière est attachée à l'utilisation de produits naturels bénéfiques pour le corps et l'environnement. Des formulations cosmétiques se revendiquant garanties d'origines naturelles ont ainsi vues le jour.

Une majorité de ces formulations ne sont en réalité pour la plupart pas à 100% d'origines naturelles, puisqu'elles contiennent des conservateurs synthétiques autorisés dans de nombreux référentiels qui certifient la naturalité. Elles peuvent de plus comprendre des allergènes, et sont souvent composées de dérivés d'huile de palme, dont l'impact de sa production sur l'environnement est très important. Ces formulations tirent classiquement leur activité d'extraits de plantes, de fruits ou de légumes, présents en concentrations souvent limitées et qui ont subi de nombreuses transformations pouvant notamment inclure des étapes agressives pour certaines molécules contenues dans lesdites plantes, fruits et légumes. Ces extraits peuvent également être sous forme d'eau obtenue par distillation des plantes, de fruits ou de légumes, qui n'est plus un gage de fraicheur des fruits, plantes ou légumes utilisés ni d'activité des molécules présentes initialement. Ces formulations sont vendues avec des périodes et des conditions de conservation larges, qui ne peuvent garantir une fraicheur absolue des ingrédients actifs malgré tous les tests engendrés. Enfin, ces compositions contiennent majoritairement de l'eau, qui le plus souvent n'est source d'aucun ingrédient actif dans la composition.

FR2961693 décrit des compositions comprenant du jus lyophilisé, qui n'est donc pas un produit frais et non transformé, et des compositions foisonnées comprenant du décyl glucoside.

FR2968551 décrit des compositions cosmétiques à base de jus de fruits, « concentrées sous vides » et « lyophilisées ».

US2018/085303 décrit des compositions cosmétiques à base de produit végétal, notamment de jus de citron, ayant subis une stérilisation.

FR3032614 décrit des compositions cosmétiques, en particulier « l'eau de citron » ayant subis une étape de distillation.

Le document Mbarki N : « Mélanger le citron et les pommes de terre pour éliminer les tâches du visage » - NewsMag - 29 octobre 2019 décrit un masque comprenant des pommes de terre et du jus de citron ou du concombre.

Il existe ainsi un besoin pour de nouvelles compositions cosmétiques qui, tout en étant réellement 100% d'origines naturelles, présentent une activité cosmétique au moins aussi importante que les compositions à base d'ingrédients d'origine synthétique, tout en préservant l'environnement et en respectant les peaux sensibles.

Aujourd'hui, la Demanderesse innove en proposant une composition cosmétique à base de produit(s) végétal(aux) et d'ingrédients 100% d'origines naturelles, sans conservateurs synthétiques, sans huile de palme, sans ingrédients d'origine de palme, et sans allergène, pour purifier, hydrater et/ou renforcer l'éclat de la peau ainsi qu'un kit pour la fabrication d'une telle composition cosmétique, par exemple par l'utilisateur lui-même à base de jus de fruit(s) ou de légume(s) fraichement pressé(s), et un procédé pour la fabrication d'une telle composition cosmétique.

Selon un premier aspect, l'invention comprend une composition cosmétique comprenant :
a. au moins le jus d'un produit végétal, de préférence au moins le jus de deux produits végétaux, ledit jus étant non transformés et ayant été obtenu au plus tard 24 heures avant la préparation de la composition,
b. de la carraghénane et du propanediol en tant qu'agent texturant, et
c. de la lysine en tant que régulateur de pH.

Par produit végétal est notamment entendu selon l'invention un jus, de la pulpe, ou encore de la purée provenant d'une espèce végétale.

Par pulpe est entendu selon l'invention les résidus de la partie charnue de certains légumes et/ou fruits.

Par purée est entendu selon l'invention des fruits ou légumes broyés et/ou écrasés.

Par jus végétal est entendu selon l'invention notamment du jus de plantes, de préférence du jus de fruits ou du jus de légumes de plantes. Par jus de légume est entendu de préférence du jus de légumes-fruits, de légumes racines, de légumes-feuilles, ou de légumes-tiges.

La désignation de légume-fruits comprend notamment l'aubergine, l'avocat, la chayotte, la citrouille, le concombre, la courge, le piment, le poivron, la tomate, le cornichon, la courgette, le haricot vert, le pois mangetout, la papaye et/ou l'olive.

La désignation de légume-racine comprend notamment la betterave, la carotte, le céleri-rave, le cerfeuil tubéreux, le chervis, le chou-rave, le galanga, le gingembre, le ginseng, la grande bardane, l'hélianthi, l'igname, le maceron, le manioc, le navet, le panais, la patate douce, le persil, la pomme de terre, le radis, le raifort, le rutabaga, le salsifis cultivé, la scorsonère d'Espagne, et/ou la topinambour.

La désignation de légume-feuille comprend notamment l'aloe vera, les salades, les choux, les épinards, l'arroche, l'asperge, la bette, le cardon, le céleri à côtes, le cerfeuil, la ciboulette, le chénopode, le laurier-sauce, la menthe, l'oseille, le persil, la sarriette, la sauge, le thym. Il s'agit le plus souvent de pulpe de feuille fraiche d'une plante.

La désignation de légumes-tiges comprend notamment la canne à sucre, le sorgho, les tubercules telle la betterave à sucre, l'agave, le céleri, le fenouil, les crosses de fougère, la rhubarbe, et/ou la bette à carde.

De manière préférée selon l'invention la composition cosmétique comprend au moins le jus de deux produits végétaux.

De manière préférée selon l'invention, le(s)dit(s) produit(s) végétal(aux) est (sont) le jus et/ou la pulpe.

De préférence, la(es)dite(s) pulpe(s) végétale(s) est(sont) choisie(s) parmi la(es) pulpe(s) de clémentine, d'orange, d'abricot, de pêche, d'ananas, du pamplemousse, du citron, de la tomate, de groseille, de la rhubarbe, de la fraise, du kiwi, de la poire, de la pomme, du melon, du raisin de la carotte, de la carotte primeur, de la citrouille, de l'aloe vera et/ou du concombre.

De préférence, lesdits jus végétaux sont choisis parmi les jus de fruits et/ou les jus de légumes.

De manière encore plus préférée, lesdits jus de légumes sont choisis parmi les jus de légume-fruits, les jus de légume-racines, les jus de légume-feuille, et/ou les jus de légumes-tiges.

Par « non-transformé » est entendu selon l'invention que lesdits fruits et/ou légumes sont utilisés dans leur forme native, sans subir de transformation telle que chauffage, pasteurisation, congélation, concentration, avant la préparation de la composition.

En effet, les fruits et les légumes contiennent de nombreuses molécules cosmétiquement actives, dont des vitamines et antioxydants naturels, qui seront donc retrouvées dans les compositions cosmétiques selon la présente invention, d'autant plus que lesdits produits de fruit(s) ou de légume(s) n'auront pas été transformés, ces molécules étant fragiles et pouvant être dégradées par des procédés de transformation.

Par « jus de fruit ou légume frais » est entendu selon l'invention un jus obtenu d'un fruit ou légume frais, sans ajout de sucre ou de conservateur. Ce type de jus doit le plus souvent être conservé au frais et a une durée de vie limitée. La composition cosmétique selon l'invention sera donc fabriquée avec ce type de jus au plus tard 24 heures après l'obtention du jus. Les vitamines et minéraux des fruits et légumes sont particulièrement bien conservés dans ce type de jus.

L'utilisation de ces produits frais et non transformés nécessite ainsi un délai de conception court de la composition selon l'invention, afin de conserver les vitamines, antioxydants et minéraux des fruits et légumes, qui sont particulièrement bien conservées dans ce type de jus et non dégradés par des procédés de transformation, à contrario des produits non-frais et transformés. De part cette conception rapide, les vitamines, antioxydants et minéraux naturels seront donc retrouvées dans les compositions cosmétiques selon la présente invention, qui se différencient ainsi en termes de bénéfice pour l'utilisateur des autres compositions cosmétiques utilisant des produits non frais et/ou transformés.

Ce délai de conception court est au maximum d'une semaine après la production du jus de fruit frais et non transformé, de préférence au maximum 24 heures après, de manière encore plus préférée au maximum 1 heure après.

Par « agent texturant » est entendu un composé permettant d'apporter à un produit cosmétique la texture désirée, tel qu'un gélifiant, un émollient ou un émulsifiant.

De manière préférée selon l'invention, l'agent texturant est un gélifiant et de manière encore préférée un gélifiant d'origine naturelle.

Lesdits gélifiants pouvant être utilisés selon l'invention comprennent, mais ne sont pas limités à :
- Genuvisco Carraghénane CG-131 (CP Kelco) : Iota carraghénane.
- Solagum AX (Seppic) : Composition comprenant 55% en poids de gomme d'acacia et 45% en poids de gomme de xanthane
- Carbogreen E.A (Azelis) : Mélange de gomme de maïs, d'amidon, de gomme de Guar, et de Sodium carraghénane,
- Genuvisco Carraghénane CG-130 (CP Kelco) : Carraghénane
- Uniqsens SFE (CP Kelco) : Mélange de pectine, de gomme de xanthane et de carraghénane

Par gomme de maïs est entendu selon l'invention un gélifiant végétal d'origine naturelle provenant notamment de l'espèce Zea Mays.

Par amidon est entendu selon l'invention l'agent gélifiant extrait de légumineuses ou de céréales comme le maïs, le riz, l'orge ou encore le blé.

Par gomme de guar est entendu selon l'invention l'agent gélifiant obtenu par broyage des graines de *Cyamopsis tetragonolobus.*

Par carraghénane est entendu selon l'invention un gélifiant végétal d'origine naturelle provenant d'extraits d'algues rouges.

Par sodium carraghénane est entendu selon l'invention le sel du gélifiant végétal d'origine naturelle provenant d'algues rouges.

Par gomme de xanthane est entendu selon l'invention l'épaississant et gélifiant obtenu à partir de l'action de la bactérie *Xanthomonas campestris.*

Par gomme d'acacia est entendu selon l'invention l'exsudat de sève produit naturellement par l'acacia, ayant des propriétés stabilisantes et de tenue pour les compositions cosmétiques.

Par pectine est entendu selon l'invention les polyosides d'origine végétales ayant des propriétés liantes et stabilisatrices de compositions cosmétiques.

De manière préférée selon l'invention, l'agent texturant est un émulsifiant et de manière encore préférée un émulsifiant d'origine naturelle.

Lesdits émulsifiants pouvant être utilisés selon l'invention comprennent, mais ne sont pas limités à :
- Emulmetik 300 IP (Lucas Meyer) : Lécithine
- Simulgreen 18 :2 (Seppic) : Émulsifiant d'origine naturelle d'originale végétale mélangeant hydroxystearyl alcohol & hydroxystearyl glucoside, et ayant également des propriétés émollientes.
- SustOleo GMS (Inolex) : Glyceryl stearate d'origine naturelle biodégradable et sans huile de palme utilisé en tant qu'émulsifiant, et ayant également des propriétés émollientes.
- Olivatis 19 : Olive oil polyglyceryl-6 esters et phospholipides, ayant des propriétés émulsifiantes, et étant 100% d'origine naturelle.
- Olivatis 12 : Polyglyceryl-3 pentaolivate ayant des propriétés émulsifiantes, et étant 100% d'origine naturelle.
- Imwitor 375 : Glyceryl citrate/lactate/linoleate/oleate, ayant une structure similaire à la lécithine, utilisé pour ses propriétés émulsifiantes, et étant 100% d'origine naturelle.

Par lécithine, ou phosphatidylcholine, est entendu selon l'invention l'émulsifiant d'origine végétal.

Par hydroxystearyl alcohol est entendu selon l'invention l'agent émulsifiant d'origine végétale.

Par hydroxystearyl glucoside est entendu selon l'invention l'agent émulsifiant d'origine végétale.

Par olive oil polyglyceryl-6 esters est entendu selon l'invention l'agent émulsifiant et émollient d'origine végétale.

Par phospholipides sont entendus les composés provenant des membranes de cellules végétales pour leur propriété émulsifiantes.

Par polyglyceryl-3 pentaolivate est entendu selon l'invention l'agent émulsifiant d'origine végétale.

Par glyceryl citrate/lactate/linoleate/oleate est entendu selon l'invention un agent émulsifiant d'origine végétal.

Par glyceryl stearate est entendu selon l'invention l'ester d'acide gras issu d'huiles végétales et ayant des propriétés émulsifiantes.

De manière préférée selon l'invention, l'agent texturant est un émollient et de manière encore préférée un émollient d'origine naturelle.

Lesdits émollients pouvant être utilisés selon l'invention comprennent, mais ne sont pas limités à :
- Simulgreen 18 :2 (Seppic) : Emulsifiant d'origine naturelle d'originale végétale mélangeant hydroxystearyl alcohol & hydroxystearyl glucoside, et ayant également des propriétés émollientes.
- SustOleo GMS (Inolex) : Glyceryl stearate d'origine naturelle biodegradable et sans huile de palme utilisé en tant qu'émulsifiant, et ayant également des propriétés émollientes.
- Vegelight 1214 LC (Biosynthis) : Alcanes de noix de coco (Coconut alkanes) et Coco caprylate/caprate. C'est une alternative d'origine naturelle aux silicones volatiles tels que la cyclométhicone D5/D6).
- Vegelight 1820 (Biosynthis) : Coconut alkanes ou C18-21 alkane. Ce sont des alcanes ramifiés à chaîne moyenne, qui est une alternative d'origine naturelle à la diméthicone.

Par alcanes de noix de coco est entendu selon l'invention des hydrocarbures saturés provenant de la noix de coco, ayant des propriétés émollientes. On retrouve notamment parmi ceux-ci les alcanes C18-21 (mélange d'alcanes ayant de 18 à 21 carbones, et ayant des propriétés émollientes).

Par esters de noix de coco, on entend notamment le coco-caprylate/caprate, qui est une huile estérifiée, ayant des propriétés émollientes.

De manière préférée selon l'invention, ladite composition comprend de la poudre de Maranta, de préférence entre 0,1 et 3% en poids de la composition.

Par poudre de Maranta ou poudre d'« arrow-root » est entendu selon l'invention la poudre qui provient de la plante *Maranta arundinacea* et qui permet de lier ou d'épaissir les préparations cosmétiques en apportant un touché non collant.

De manière préférée selon l'invention, ladite composition comprend du tocophérol, de préférence entre 0,1 et 0,5% en poids de la composition.

Par tocophérol est entendu selon l'invention la vitamine E, connue notamment pour ses propriétés anti-oxydantes.

De manière préférée, la composition comprend 0,1 à 2 % de lysine en poids de la composition finale.

De manière préférée, la composition comprend en outre au moins un autre régulateur de pH, choisi parmi le carbonate de calcium, l'acide citrique et/ou le jus de citron.

De manière préférée selon l'invention, ledit au moins un autre régulateur de pH,est présent entre 0,1 à 2 % en poids de la composition finale.

Par régulateur de pH est entendu un élément permettant de moduler (réduire ou augmenter) le pH de la phase aqueuse autour de 5 à 5,5. La lysine, le carbonate de calcium, l'acide citrique et/ou le jus de citron sont utilisés ici en tant que régulateur de pH.

De manière préférée selon l'invention, le(s)dit(s) jus végétal(aux) est(sont) choisi(s) parmi le(s) jus de clémentine, d'orange, d'abricot, de pêche, d'ananas, du pamplemousse, du citron, de la tomate, de groseille, de la rhubarbe, de la fraise, du kiwi, de la poire, de la pomme, du melon, du raisin de la carotte, de la carotte primeur, de la citrouille, de l'aloe vera et/ou du concombre.

De manière préférée les compositions selon l'invention comprennent le mélange d'au moins deux jus végétaux, et de manière encore préférée les mélanges sont :
- Clémentine - Carotte - Orange
- Carotte - Orange
- Carotte primeur - Abricot - Pêche
- Carotte - Abricot - Pêche
- Pamplemousse - Citron - Citrouille
- Pamplemousse - Citrouille
- Pamplemousse - Rhubarbe
- Pamplemousse - Rhubarbe - Fraise
- Pamplemousse - Tomate - Groseille - Rhubarbe - Fraise
- Pamplemousse - Citron - Tomate - Rhubarbe - Fraise
- Pamplemousse - Citron - Tomate - Rhubarbe - Citrouille
- Kiwi - Poire - Aloe vera - Pomme
- Poire - Aloe vera - Pomme
- Aloe vera - Concombre
- Aloe vera - Concombre - Melon
- Poire - Aloe vera - Pomme - Concombre - Melon
- Poire - Aloe vera - Pomme - Raisin
- Pamplemousse - Tomate - Fraise
- Pêche - Orange - Ananas
- Raisin - Melon - Concombre
- Tomate - Fraise
- Pomme - Raisin
- Orange - Ananas

De manière encore préférée selon l'invention, lesdits mélanges d'au moins deux jus végétaux sont réalisés de la manière suivante :
- 1/3 en poids du mélange de jus de pêche, 1/3 en poids du mélange de jus d'orange, et 1/3 en poids du mélange de jus d'ananas, ou
- 37,5% en poids du mélange de jus de raisin, 37,5% en poids du mélange de jus de melon, et 25% en poids du mélange de jus de concombre, ou
- 50% en poids du mélange de jus de pamplemousse, 20% en poids du mélange de jus de tomate, et 30% en poids du mélange de jus de fraise, ou
- 1/2 en poids du mélange de jus d'orange, 1/2 en poids du mélange de jus d'ananas ,ou
- 70% en poids du mélange de jus de pamplemousse, 30 % en poids du mélange de jus de tomate

L'homme du métier, formulateur cosmétique, s'assurera grâce à ses connaissances générales de choisir des végétaux et en particulier de fruit(s) et/ou légumes(s) permettant d'obtenir un produit ayant une quantité d'eau suffisante, sans eau ajoutée, pour permettre la fabrication d'une composition cosmétique selon l'invention et avec les caractéristiques galéniques voulues.

De manière préférée selon l'invention, lesdits végétaux mélangés sont sélectionnés en fonction de leur période de récolte. Le(s) produit(s) végétal(aux) étant préférentiellement frais et/ou non transformés, avec un impact le plus limité possible sur l'environnement, sera (seront) sélectionné(s) de manière préférée et dans la mesure du possible parmi des fruits et/ou légumes de saison, c'est à dire poussant naturellement dans la zone de fabrication, et lors du mois de fabrication de ladite composition cosmétique.

De manière préférée selon l'invention, le(s)dit(s) produits végétal(aux) apporte(nt) la totalité de l'eau nécessaire à l'obtention de la composition finale.

De manière préférée selon l'invention, la composition comprend 90%, de manière encore préférée 95%, de manière encore plus préférée 99%, de manière particulièrement préférée 100% en poids de composants d'origine naturelle.

De manière préférée selon l'invention, lesdites compositions ne comprennent pas d'ingrédients d'origine de palme. En particulier, lesdites compositions ne comprennent pas d'huile de palme.

De manière préférée selon l'invention, la composition obtenue est sous forme de gel, de sérum, de masque, de lotion, de crème, de pommade, d'onguent, de masque gel ou d'émulsions huile dans eau ou eau dans huile.

De manière préférée selon l'invention, ladite composition comprend :

| Phase | Nom commercial | Nom INCI | Société | Rôle | % |
|---|---|---|---|---|---|
| A | JUS PAMPLEMOUSS E, TOMATE, FRAISE (PROPORTIONS 50% PAMPLEMOUSS E, 20% TOMATE, 30% FRAISE) | - | GREENTE CH | - | QSP 100 |
| | PROPANEDIOL | PROPANEDIO L | CONNECT CHEMICAL | SOLVANT | 12-18 |
| | LYSINE | LYSINE | EMEISHAN LONGTEN G BIOTECHN OLOGY Co., Ltd | REGULATEU R PH | 0,1-2 |
| | UNIQSENS SFE SYSTEM | XANTHAN GUM AND PECTINE AND CARRAGEEN AN | CP KELCO | GELIFIANT | 0,1-2 |
| | GENUVSICO CARRAGEENAN CG- 131 | CARRAGEEN AN | CP KELCO | GELIFIANT | 0,1-2 |
| B | SUSTOLEO GMS | GLYCERYL STEARATE | INOLEX | CO-EMULSIFIER | 1-5 |
| | SIMULGREEN 18 :2 | HYDROXYST EARYL ALCOHOL (AND) HYDROXYST EARYL GLUCOSIDE | SEPPIC | EMULSIFIER | 1-5 |
| | VEGELIGHT 1214 LC | COCONUT ALKANES (AND) COCO-CAPRYLATE/ CAPRATE | BIOSYNTHI S | EMOLLIENT, SILICONE LIKE | 2-8 |
| | VEGELIGHT 1820 | COCONUT ALCANES | BIOSYNTHI S | EMOLLIENT, SILICONE LIKE | 2-8 |
| | POUDRE DE MARANTA BIO (911009) | MARANTA ARUNDINACE A ROOT POWDER | GREENTE CH | MATIFIANT | 0,5-3 |
| C | COVIOX T70C | TOCOPHERO L | BASF | ANTIOXYDAN T | 0,1-0,5 |

De manière préférée selon l'invention, ladite composition comprend :

| Phase | Nom commercial | Nom INCI | Société | Rôle | % |
|---|---|---|---|---|---|
| A | JUS RAISIN, MELON, CONCOMBRE (PROPORTION S 25% CONCOMBRE, 37,5% RAISIN, 37,5% MELON) | - | GREENTEC H | - | QSP 100 |
| | PROPANEDIOL | PROPANEDIOL | CONNECT CHEMICAL | SOLVANT | 12-18 |
| | LYSINE | LYSINE | EMEISHAN LONGTENG BIOTECHNO LOGY Co., Ltd | REGULATEUR PH | 0,1-2 |
| | GENUVSICO CARRAGEENA N CG- 131 | CARRAGEENAN GUM | CP KELCO | GELIFIANT | 0,1-1 |
| | SOLAGUM AX | ACACIA SNEGAL GUM & XANTHAN GUM | SEPPIC | GELIFIANT | 0,1-1 |
| | EMULMETIK 300 IP | LECITHIN | LUCAS MEYER | GELIFIANT/EM ULSIONNANT | 0,1-1 |
| | VEGELIGHT 1820 | COCONUT ALKANES | BIOSYNTHIS | EMOLLIENT | 0,1-2 |
| | POUDRE DE MARANTA BIO (911009) | MARANTA ARUNDINACEA ROOT POWDER | GREENTEC H | MATIFIANT | 0,1-2 |

De manière préférée selon l'invention, ladite composition comprend :

| Phase | Nom commercial | Nom INCI | Société | Rôle | % |
|---|---|---|---|---|---|
| A | JUS RAISIN MELON CONCOMBRE (PROPORTIONS 25% concombre, 37,5% melon, 37,5% raisin) | - | GREENTEC H | - | QSP 100 |
| | PROPANEDIOL | PROPANEDIOL | CONNECT CHEMICAL | SOLVANT | 12-18 |
| | CARBOGREEN E.A | ZEA MAYS (CORN) STRACH (AND) CYAMOPSIS TETRAGONOLO BA (GUAR) GUM (AND) SODIUM CARRAGEENAN | AZELIS | GELIFIANT | 0,5-2 |
| | GENUVISCO CARRAGEENAN CG-130 | CARRAGEENAN GUM | CP KELCO | GELIFIANT | 0,2-1 |
| | GENUVISCO CARRAGEENAN CG-131 | CARRAGEENAN GUM | CP KELCO | GELIFIANT | 0,1-1 |

De manière préférée selon l'invention, ladite composition comprend :

| Phase | Nom commercial | Nom INCI | Société | Rôle | % |
|---|---|---|---|---|---|
| A | JUS ORANGE | - | GREENTEC H | - | QSP 100 |
| | PROPANEDIO L | PROPANEDIOL | CONNECT CHEMICAL | SOLVANT | 12-18 |
| | LYSINE | LYSINE | EMEISHAN LONGTENG BIOTECHNO LOGY Co., Ltd | REGULATEUR PH | 0,1-2 |
| | GENUVSICO CARRAGHÉN ANE CG- 131 | CARRAGHÉNANE GUM | CP KELCO | GELIFIANT | 0,1-1 |
| | SOLAGUM AX | ACACIA SNEGAL GUM & XANTHAN GUM | SEPPIC | GELIFIANT | 0,1-1 |
| | EMULMETIK 300 IP | LECITHIN | LUCAS MEYER | GELIFIANT/EM ULSIONNANT | 0,1-1 |
| | VEGELIGHT 1820 | COCONUT ALKANES | BIOSYNTHIS | Emollient | 0,1-2 |
| | POUDRE DE MARANTA BIO (911009) | MARANTA ARUNDINACEA ROOT POWDER | GREENTEC H | MATIFIANT | 0,1-2 |

De manière préférée selon l'invention, ladite composition comprend :

| Phase | Nom commercial | Nom INCI | Société | Rôle | % |
|---|---|---|---|---|---|
| A | JUS ORANGE, ANANAS (PROPORTION 50/50) | | GREENTEC H | | QSP 100 |
| | PROPANEDIOL | PROPANEDIOL | CONNECT CHEMICAL | SOLVANT | 12-18 |
| | LYSINE | LYSIN | EMEISHAN LONGTENG BIOTECHNO LOGY Co., Ltd | REGULATEUR PH | 0,1-2 |
| | GENUVSICO CARRAGHÉNA NE CG- 131 | CARRAGHÉNANE GUM | CP KELCO | GELIFIANT | 0,1-1 |
| | SOLAGUM AX | ACACIA SNEGAL GUM & XANTHAN GUM | SEPPIC | GELIFIANT | 0,1-1 |
| | EMULMETIK 300 IP | LECITHIN | LUCAS MEYER | GELIFIANT/EM ULSIONNANT | 0,1-1 |
| | VEGELIGHT 1820 | COCONUT ALKANES | BIOSYNTHIS | Emollient | 0,1-2 |
| | POUDRE DE MARANTA BIO (911009) | MARANTA ARUNDINACEA ROOT POWDER | GREENTEC H | MATIFIANT | 0,1-2 |

De manière préférée selon l'invention, ladite composition comprend :

| Phase | Nom commercial | Nom INCI | Société | Rôle | % |
|---|---|---|---|---|---|
| | PULPE D'ORANGE | | GRRENTEC H | | QSP 100 |
| A | PROPANEDIOL | PROPANEDIOL | CONNECT CHEMICAL | SOLVANT | 12-18 |
| | LYSINE | LYSINE | EMEISHAN LONGTENG BIOTECHNO LOGY Co., Ltd | REGULATEUR PH | 0,1-2 |
| | GENUVISCO CARRAGEENAN CG-131 | CARRAGEENAN GUM | CP KELCO | GELIFIANT | 0,1-1 |

Selon un deuxième aspect, l'invention consiste en un procédé de fabrication d'une composition cosmétique, comprenant au moins une étape de mélange de carraghénane et de propanediol en tant qu'agents texturants, avec au moins le jus d'un produit végétal, non transformés et ayant été obtenu au plus tard 24 heures avant la préparation de la composition, et avec de la lysine en tant que régulateur de pH.

De manière préférée, le procédé comprend l'ajout d'entre 0,1 à 2 % de lysine en poids de la composition finale.

De manière préférée selon l'invention, ledit procédé comprend une étape d'ajout de poudre de Maranta, de préférence entre 0,1 et 3% en poids de la composition.

De manière préférée selon l'invention, ledit procédé comprend une étape d'ajout de tocophérol, de préférence entre 0,1 et 0,5% en poids de la composition.

De manière préférée selon l'invention, ledit procédé comprend une étape d'ajout d'un autre régulateur de pH, de préférence choisi le carbonate de calcium, l'acide citrique et/ou le jus de citron, de manière encore plus préférée entre 0,1 à 2 % en poids de la composition finale.

De manière préférée selon l'invention, le(s) produit(s) végétal(aux) est (sont) obtenus par pressage, centrifugation, broyage ou extraction. De manière encore préférée l'étape de pressage, centrifugation, broyage ou extraction est effectuée à froid, sans étape de chauffage.

De manière préférée selon l'invention, une fois obtenu, le(s) produit(s) végétal(aux) est filtré ou tamisé afin de filtrer la pulpe, et de récupérer le jus.

De manière préférée selon l'invention, le(s)dit(s) produit végétal(aux) est (sont) obtenu(s) par des étapes de
a. Pressage sans chauffage des éléments désirés de la plante, de préférence les fruits et/ou légumes et/ou racines et/ou tiges et/ou feuilles de la plante, afin d'en extraire le jus ;
b. Passage sur un tamis dudit jus afin de récupérer les produits végétaux desdits fruits : le jus sans pulpe après passage sur le tamis et la pulpe restée sur tamis.

De manière préférée, le mélange de(s)dit(s) produit végétal(aux) frais et/ou non transformés), obtenu(s) par les étapes de pressage et de passage sur un tamis, avec ledit au moins un agent texturant est réalisé au maximum une semaine après l'obtention desdits produits végétaux, de préférence au maximum 24 heures après, de manière encore plus préférée au maximum 1 heure après.

De manière préférée selon l'invention, ledit procédé comprend au moins deux produits végétaux, de préférence non transformés et ayant été obtenu au plus tard 24 heures avant la préparation de la composition.

De manière préférée selon l'invention, dans le cas où la composition cosmétique comprend au moins deux fruit(s) et/ou légume(s) différents, lesdits fruits ou légumes sont préférentiellement pressés et passés sur tamis séparément puis les jus ainsi obtenus sont mélangés dans une étape c. avant mélange avec l'agent texturant.

De manière préférée selon l'invention, dans le cas où la composition cosmétique comprend au moins deux fruit(s) et/ou légume(s) différents, lesdits fruits ou légumes sont préférentiellement pressés et passés sur tamis séparément puis les pulpes ainsi obtenues sont mélangées dans une étape c. avant mélange avec l'agent texturant.

De manière préférée selon l'invention, ladite étape c. peut consister en un mélange de pulpe avec du jus.

De manière préférée selon l'invention, ledit mélange est réalisé sous agitation, de préférence entre 15°C et 90°C, de préférence entre 20 et 60°C, et de manière tout particulièrement préférée à température ambiance entre 20 et 30°C. En effet, il sera particulièrement recherché de réaliser le procédé à une température ambiante, afin de conserver les vitamines et minéraux des fruits et légumes.

De manière préférée selon l'invention, ledit chauffage dure entre 1 et 5 minutes.

De manière préférée, lesdits agents texturants sont mélangés avec entre 30 et 95%, de préférence entre 65 et 85%, en poids de la composition finale d'au moins un produit végétal, non transformés et ayant été obtenu au plus tard 24 heures avant la préparation de la composition.

De manière préférée selon l'invention, lorsque ladite composition est formulée sous forme d'émulsion, ladite composition comprend entre 60 et 95%, de préférence entre 65 et 82%, en poids de la composition finale d'au moins un produit végétal, de préférence non transformés et ayant été obtenu au plus tard 24 heures avant la préparation de la composition.

De manière préférée selon l'invention, lorsque ladite composition est formulée sous forme de masque gel, ladite composition comprend entre 45 et 90%, de préférence entre 68 et 83%, en poids de la composition finale d'au moins un produit végétal, de préférence non transformés et ayant été obtenu au plus tard 24 heures avant la préparation de la composition.

De manière préférée selon l'invention, lorsque ladite composition est formulée sous forme de sérum, ladite composition comprend entre 70 et 90%, de préférence entre 78 et 85%, en poids de la composition finale d'au moins un produit végétal, de préférence non transformés et ayant été obtenu au plus tard 24 heures avant la préparation de la composition.

De manière préférée selon l'invention, dans le cas où les agents texturants consistent en du propanediol, de la pectine, de la gomme de xanthane, de la carraghénane, du glyceryl stearate, de l'hydroxystearyl alcohol, de l'hydroxystearyl glucoside, de l'alcane de noix de coco, des esters de noix de coco, ceux-ci sont mélangés avec entre 60 et 95%, de préférence entre 65 et 82%, en poids de la composition finale d'au moins un produit végétal, de préférence non transformés et ayant été obtenu au plus tard 24 heures avant la préparation de la composition.

De manière préférée selon l'invention, ledit mélange comprend entre 10 et 20%, de préférence 15%, en poids de la composition finale de propanediol, entre 0,50 et 0,75%, de préférence 0,65%, en poids de la composition finale d'un mélange de pectine, de gomme de xanthane et de carraghénane, entre 2 et 4%, de préférence 3%, en poids de la composition finale d'un mélange de hydroxystearyl alcohol et de hydroxystearyl glucoside , entre 4 et 6%, de préférence 5%, en poids de la composition finale d'un mélange d'alcane de noix de coco et d'esters de noix de coco.

De manière préférée selon l'invention, dans le cas où les agents texturants consistent en du propanediol, de la carraghénane, de la gomme de xanthane, de la lécithine, et de l'alcane de noix de coco, ceux-ci sont mélangés avec entre 45 et 90%, de préférence entre 68 et 83%, en poids de la composition finale d'au moins un produit végétal, de préférence non transformés et ayant été obtenu au plus tard 24 heures avant la préparation de la composition.

De manière préférée selon l'invention, ledit mélange comprend entre 10 et 20%, de préférence 15%, en poids de la composition finale de propanediol, entre 0,1 et 0,3%, de préférence 0,2%, en poids de la composition finale de carraghénane, entre 0,05 et 0,15%, de préférence 0,1%, en poids de de la composition finale de gomme de xanthane, entre 0,1% et 0,3%, de préférence 0,2%, en poids de la composition finale de lécithine, et entre 1 et 1,6%, de préférence 1,3%, en poids de la composition finale d'alcanes de noix de coco.

De manière préférée selon l'invention, dans le cas où les agents texturants consistent en du propanediol, de la carraghénane, de la gomme de maïs, de l'amidon, de la gomme de guar, et du sodium carraghénane, ceux-ci sont mélangés avec entre 70 et 90%, de préférence entre 78 et 85%, en poids de la composition finale d'au moins un produit végétal, de préférence non transformés et ayant été obtenu au plus tard 24 heures avant la préparation de la composition.

De manière préférée selon l'invention, ledit mélange comprend entre 10 et 20%, de préférence 15%, en poids de la composition finale de propanediol, entre 0,1 et 0,3%, de préférence 0,2%, en poids de la composition finale de carraghénane, et entre 0,05 et 0,15%, de préférence 0,1%, en poids d'un mélange de gomme de maïs, d'amidon, de gomme de guar, et de sodium carraghénane.

Selon un troisième aspect, l'invention consiste en l'utilisation de la composition cosmétique selon l'invention pour améliorer l'aspect de la peau et en particulier purifier et/ou hydrater et/ou renforcer l'éclat de la peau après application topique.

De manière préférée, la composition cosmétique selon l'invention est appliquée sur la peau du visage.

Selon un quatrième aspect, l'invention consiste en un kit pour la fabrication d'une composition cosmétique caractérisé en ce qu'il comprend :
a. Au moins le jus d'un produit végétal, non transformé et ayant été obtenu au plus tard 24 heures avant la préparation de la composition ;
b. De la carraghénane et du propanediol, en tant qu'agent texturant,
c. De la lysine en tant que régulateur de pH ;
d. Optionnellement du tocophérol ;
e. Optionnellement de la poudre de Maranta.

De manière préférée, le kit selon la présente invention comprend un autre régulateur de pH choisi parmi le carbonate de calcium, l'acide citrique et/ou le jus de citron.

De manière préférée, le kit selon l'invention comprend au moins deux produits végétaux, de préférence non transformés et ayant été obtenu au plus tard 24 heures avant la préparation de la composition.

Selon un mode de réalisation de l'invention, l'utilisateur peut presser un ou plusieurs fruit(s) et/ou légume(s), par exemple à son domicile ou sur un point de vente, puis le mélanger à un agent texturant ou une association d'agent texturant et optionnellement un régulateur de pH, pour obtenir une composition cosmétique personnalisée et prête à l'emploi.

### Exemples :

### Exemple 1 : Préparation des produits végétaux.

Le ou les fruits et/ou légumes sont pressés indépendamment, sans chauffage, afin d'en extraire le jus.

Le(s) jus récupéré(s) est (sont) ensuite passé(s) sur tamis afin de filtrer la pulpe, et de récupérer le jus.

Suivant les formulations, les jus ou les pulpes récupérés sont mélangés en différentes proportions.

### Exemple 2 : Procédé d'obtention d'une composition cosmétique sous forme d'émulsion.

### (Phase A)

La quantité nécessaire de jus ou de pulpe est mélangée sous faible agitation avec entre 12 et 18% de propanediol.

Entre 0,1 et 2% de lysine est ajouté afin de remonter le pH de la phase aqueuse autour de 5-5,5.

Il est ensuite ajouté sous agitation :
- entre 0,1 et 2% en poids de la composition finale d'un mélange de pectine, de gomme de xanthane et de Carraghénane (Uniqsens SFE (CP Kelco)) ; et
- entre 0,1 et 2% en poids de la composition finale de Carraghénane (Genuvisco Carraghénane CG-131 (CP Kelco))

La composition est mélangée jusqu'à obtenir un gel homogène.

La composition est ensuite chauffée à 75°C pendant 5 minutes.

### (Phase B)

Dans un autre récipient sont mélangés :
- entre 1 et 5% en poids de la composition finale d'un mélange de Hydroxystearyl Alcohol & Hydroxystearyl Glucoside (Simulgreen 18 :2 (Seppic)) ;
- entre 1 et 5% en poids de la composition finale de Glyceryl stearate (SustOleo GMS (Inolex))
- entre 2 et 8% en poids de la composition finale d'un mélange d'alcanes et d'esters de noix de coco (Vegelight 1214 LC (Biosynthis))
- entre 2 et 8% en poids de la composition finale d'alcanes de noix de coco (Vegelight 1820 (Biosynthis))
- entre 0,5 et 3% en poids de la composition finale de Poudre de Maranta bio Ce mélange est chauffé à 75°C pendant 2 minutes.

La phase A et la phase B sont ensuite mélangées sous vive agitation pendant 5 minutes.

Le mélange obtenu est laissé refroidir jusqu'à 30°C sous agitation modérée, et il est ajouté entre 0,1 et 0,5% en poids de la composition finale de tocophérol.

### Exemple 3 : Procédé d'obtention d'une composition cosmétique sous forme de sérum, à chaud.

La quantité nécessaire de jus ou de pulpe est mélangée sous faible agitation à entre 12 et 18% en poids de la composition finale de propanediol.

Entre 0,1 et 2% en poids de la composition finale de lysine est ajouté afin de remonter le pH de la phase aqueuse autour de 5-5,5.

Il est ensuite ajouté sous agitation :
- entre 0,1 et 2% en poids de la composition finale de lécithine (EMULMETIK 300 IP) ;
- entre 0,1 et 2% en poids de la composition finale de Carraghénane (Genuvisco Carraghénane CG-131 (CP Kelco))
- entre 0,1 et 2% en poids de la composition finale d'un mélange comprenant 55% en poids de gomme d'acacia et 45% en poids de gomme de xanthane (Solagum AX (Seppic))

La composition est chauffée à 65°C pendant 5 minutes et mélangée jusqu'à obtenir un gel homogène.

Il est ensuite ajouté entre 0,1 et 2% en poids de la composition finale d'alcanes de noix de coco (Vegelight 1820 (Biosynthis)) et la composition est mélangée pendant 5 minutes sous vive agitation.

La composition est laissée refroidir jusqu'à 30°C sous agitation.

Entre 0,1 et 3% en poids de la composition finale de Poudre de Maranta bio est enfin ajouté.

### Exemple 4 : Procédé d'obtention d'une composition cosmétique sous forme de sérum, à froid.

La quantité nécessaire de jus ou de pulpe est mélangée sous faible agitation à entre 12 et 18% en poids de la composition finale de propanediol.

Entre 0,1 et 2% en poids de la composition finale de lysine est ajouté afin de remonter le pH de la phase aqueuse autour de 5-5,5.

Il est ensuite ajouté sous agitation :
- entre 0,1 et 2% en poids de la composition finale de lécithine (EMULMETIK 300 IP) ;
- entre 0,1 et 2% en poids de la composition finale de Carraghénane (Genuvisco Carraghénane CG-131 (CP Kelco))
- entre 0,1 et 2% en poids de la composition finale d'un mélange comprenant 55% en poids de gomme d'acacia et 45% en poids de gomme de xanthane (Solagum AX (Seppic))

La composition est mélangée jusqu'à obtenir un gel homogène.

Il est ensuite ajouté entre 0,1 et 2% en poids de la composition finale d'alcanes de noix de coco (Vegelight 1820 (Biosynthis)) et la composition est mélangée pendant 5 minutes sous vive agitation.

Entre 0,1 et 3% en poids de la composition finale de Poudre de Maranta bio est enfin ajouté.

### Exemple 5 : Procédé d'obtention d'une composition cosmétique sous forme d'un masque gel :

La quantité nécessaire de jus ou de pulpe est mélangée sous faible agitation à entre 12 et 18% en poids de la composition finale de propanediol.

Entre 0,1 et 2% en poids de la composition finale de lysine est ajouté afin de remonter le pH de la phase aqueuse autour de 5-5,5.

La composition est chauffée à 90°C pendant 5 minutes sous agitation.

Il est ensuite ajouté sous agitation :
- entre 0,5 et 2% en poids de la composition finale d'un mélange de gomme de maïs, d'amidon, de gomme de Guar, et de Sodium carraghénane, (Carbogreen E.A (Azelis))
- entre 0,2 et 1% en poids de la composition finale de Carraghénane (Genuvisco Carraghénane CG-130 (CP Kelco))
- entre 0,1 et 1% en poids de la composition finale de Carraghénane (Genuvisco Carraghénane CG-131 (CP Kelco))

La composition est mélangée sous forte agitation jusqu'à obtenir un mélange homogène.

La composition est laissée refroidir jusqu'à 30°C sous agitation.

## Revendications

1. Composition cosmétique comprenant :
a. au moins le jus d'un produit végétal, de préférence au moins le jus de deux produits végétaux, ledit jus étant non transformés et ayant été obtenu au plus tard 24 heures avant la préparation de la composition,
b. de la carraghénane et du propanediol en tant qu'agent texturant, et
c. de la lysine en tant que régulateur de pH.

2. Composition selon la revendication précédente, comprenant 0,1 à 2 % de lysine en poids de la composition finale.

3. Composition selon l'une quelconque des revendications précédentes, comprenant en outre au moins un autre régulateur de pH, choisi parmi le carbonate de calcium, l'acide citrique et/ou le jus de citron.

4. Composition selon l'une quelconque des revendications précédentes, **caractérisé en ce que** le(s) produits végétal(aux) apporte(nt) la totalité de l'eau nécessaire à l'obtention de la composition finale

5. Procédé de fabrication d'une composition cosmétique, comprenant au moins une étape de mélange de carraghénane et de propanediol en tant qu'agents texturants, avec au moins le jus d'un produit végétal, non transformés et ayant été obtenu au plus tard 24 heures avant la préparation de la composition, et avec de la lysine en tant que régulateur de pH.

6. Procédé selon la revendication précédente, comprenant entre 0,1 à 2 % de lysine en poids de la composition finale.

7. Procédé selon les revendications 5 et 6, **caractérisé en ce que** le(s)dit(s) produit végétal(aux) est (sont) obtenu(s) par des étapes de :
a. Pressage sans chauffage des éléments désirés de la plante, de préférence les fruits et/ou légumes et/ou racines et/ou tiges et/ou feuilles de la plante, afin d'en extraire le jus ;
b. Passage sur un tamis dudit jus afin de récupérer les produits végétaux desdits fruits : le jus sans pulpe après passage sur le tamis et la pulpe restée sur tamis.

8. Procédé selon les revendications 5 à 7, **caractérisé en ce que** lesdits agents texturants sont mélangés avec entre 30 et 95%, de préférence entre 65 et 85%, en poids de la composition finale d'au moins un produit végétal, non transformés et ayant été obtenu au plus tard 24 heures avant la préparation de la composition.

9. Utilisation de la composition cosmétique selon les revendications 1 à 4 pour purifier et/ou hydrater et/ou renforcer l'éclat de la peau après application topique.

10. Kit pour la fabrication d'une composition cosmétique **caractérisé en ce qu'**il comprend :
a. Au moins le jus d'un produit végétal, non transformé et ayant été obtenu au plus tard 24 heures avant la préparation de la composition ;
b. De la carraghénane et du propanediol, en tant qu'agent texturant,
c. De la lysine en tant que régulateur de pH ;
d. Optionnellement du tocophérol ;
e. Optionnellement de la poudre de Maranta.

## Patentansprüche

1. Kosmetische Zusammensetzung, umfassend:
a. mindestens den Saft eines pflanzlichen Produkts, vorzugsweise mindestens den Saft von zwei pflanzlichen Produkten, wobei der Saft unverarbeitet ist und spätestens 24 Stunden vor der Herstellung der Zusammensetzung gewonnen wurde,
b. Carrageen und Propandiol als Texturierungsmittel, und
c. Lysin als pH-Regler.

2. Zusammensetzung nach dem vorstehenden Anspruch, umfassend 0,1 bis 2 % Lysin, bezogen auf das Gewicht der fertigen Zusammensetzung.

3. Zusammensetzung nach einem der vorstehenden Ansprüche, ferner umfassend mindestens einen weiteren pH-Regler, ausgewählt aus Calciumcarbonat, Citronensäure und/oder Zitronensaft.

4. Zusammensetzung nach einem der vorstehenden Ansprüche,
**dadurch gekennzeichnet, dass** das/die pflanzliche(n) Produkt(e) das gesamte Wasser liefert/liefern, das für den Erhalt der fertigen Zusammensetzung erforderlich ist.

5. Verfahren zur Herstellung einer kosmetischen Zusammensetzung, umfassend mindestens einen Schritt des Mischens von Carrageen und Propandiol als Texturierungsmittel mit mindestens dem Saft eines pflanzlichen Produkts, der unverarbeitet ist und spätestens 24 Stunden vor der Herstellung der Zusammensetzung gewonnen wurde, und mit Lysin als pH-Regler.

6. Verfahren nach dem vorstehenden Anspruch, umfassend zwischen 0,1 und 2 % Lysin, bezogen auf das Gewicht der fertigen Zusammensetzung.

7. Verfahren nach den Ansprüchen 5 und 6,
**dadurch gekennzeichnet, dass** das/die pflanzliche(n) Produkt(e) durch folgende Schritte erhalten wird/werden:
a. Pressen ohne Erhitzen der gewünschten Elemente der Pflanze, vorzugsweise der Früchte und/oder Gemüse und/oder Wurzeln und/oder Stängel und/oder Blätter der Pflanze, um den Saft daraus zu extrahieren;
b. Passieren des Saftes durch ein Sieb, um die pflanzlichen Produkte der Früchte zu gewinnen: den fruchtfleischfreien Saft nach dem Passieren durch das Sieb und das auf dem Sieb verbliebene Fruchtfleisch.

8. Verfahren nach den Ansprüchen 5 bis 7, **dadurch gekennzeichnet, dass** die Texturierungsmittel mit zwischen 30 und 95 Gew.-%, vorzugsweise zwischen 65 und 85 Gew.-%, der fertigen Zusammensetzung mindestens eines pflanzlichen Produkts gemischt werden, das unverarbeitet ist und spätestens 24 Stunden vor der Herstellung der Zusammensetzung erhalten wurde.

9. Verwendung der kosmetischen Zusammensetzung nach den Ansprüchen 1 bis 4 zum Reinigen und/oder Hydratisieren und/oder Verstärken der Ausstrahlung der Haut nach topischer Anwendung.

10. Kit zur Herstellung einer kosmetischen Zusammensetzung,
**dadurch gekennzeichnet, dass** es umfasst:
a. mindestens den Saft eines pflanzlichen Produkts, der unverarbeitet ist und spätestens 24 Stunden vor der Herstellung der Zusammensetzung erhalten wurde;
b. Carrageen und Propandiol als Texturierungsmittel,
c. Lysin als pH-Regler;
d. gegebenenfalls Tocopherol;
e. gegebenenfalls Maranta-Pulver.

## Claims

1. A cosmetic composition comprising:
a. at least the juice of one plant product, preferably at least the juice of two plant products, said juice being unprocessed and having been obtained no later than 24 hours before the preparation of the composition,
b. carrageenan and propanediol as texturizing agents, and
c. lysine as a pH regulator.

2. The composition according to the preceding claim, comprising 0.1 to 2% lysine by weight of the final composition.

3. The composition according to any of the preceding claims, further comprising at least one other pH regulator selected from calcium carbonate, citric acid, and/or lemon juice.

4. The composition according to any of the preceding claims, **characterized in that** the plant product(s) provide(s) all of the water required to obtain the final composition

5. A method for manufacturing a cosmetic composition, comprising at least one step of mixing carrageenan and propanediol as texturizing agents, with at least the juice of a plant product, unprocessed and having been obtained no later than 24 hours before the preparation of the composition, and with lysine as a pH regulator.

6. The method according to the preceding claim, comprising between 0.1 and 2% lysine by weight of the final composition.

7. The method according to claims 5 and 6, **characterized in that** said plant product(s) is (are) obtained through the following steps:
a. Cold-pressing the desired parts of the plant, preferably the fruits and/or vegetables and/or roots and/or stems and/or leaves of the plant, to extract the juice therefrom;
b. Passing said juice through a sieve to recover the plant products from said fruits: the juice without pulp after passing through the sieve and the pulp remaining on the sieve.

8. The method according to claims 5 to 7, **characterized in that** said texturizing agents are mixed with between 30 and 95%, preferably between 65 and 85%, by weight of the final composition of at least one plant product, unprocessed and having been obtained no later than 24 hours before the preparation of the composition.

9. A use of the cosmetic composition according to claims 1 to 4, to purify and/or moisturize and/or enhance the radiance of the skin after topical application.

10. A kit for the manufacture of a cosmetic composition, **characterized in that** it comprises:
a. At least the juice of one plant product, unprocessed and having been obtained no later than 24 hours before the preparation of the composition;
b. Carrageenan and propanediol, as texturizing agents,
c. Lysine as a pH regulator;
d. Optionally, tocopherol;
e. Optionally, Maranta powder.
